# EUROPEAN PATENT SPECIFICATION

(11) **EP 2 329 801 B1**
(45) Date of publication and mention of the grant of the patent: **22.03.2017**
(21) Application number: 09814697.0
(22) Date of filing: 18.09.2009
(51) Int. Cl.: A61F 5/44, A61F 5/451, A61F 5/455, A61F 13/15, G01M 3/40, A61F 13/42

(54) **EXCRETION SENSING DEVICE**
VORRICHTUNG ZUR ERKENNUNG VON FLÜSSIGKEITSAUSTRITTEN
DISPOSITIF DE DÉTECTION DE LIQUIDE D'EXCRÉTION

(30) Priority: 19.09.2008 JP 2008241777
(43) Date of publication of application: 08.06.2011
(73) Proprietor: Unicharm Corporation, Ehime 799-0111 (JP)
(72) Inventor: WADA, Ichiro, Kanonji-shi Kagawa 769-1602 (JP); SUZUKI, Miou, Kanonji-shi Kagawa 769-1602 (JP)
(74) Representative: Dolleymores
(86) International application number: PCT/JP2009/066454
(87) International publication number: WO 2010/032853

(56) References cited:
- EP-A1- 1 616 542
- WO-A1-2008/001475
- JP-A- 9 294 762
- JP-A- 2002 055 074
- JP-A- 2006 026 108
- JP-A- 2007 044 493
- US-A1- 2007 038 194
- US-A1- 2007 083 174
- US-B1- 6 200 250

## Description

### TECHNICAL FIELD

The present invention relates to excretion sensing devices and more particularly to excretion sensing devices including an excretion sensor adapted to sense excretions such as urine.

### RELATED ART

Conventionally, wearing articles such as pants provided on the side facing the wearer's body with a urine sensor are known, for example, from PATENT DOCUMENT 1 listed below. According to the disclosure of PATENT DOCUMENT 1, a urine sensor is sandwiched between a pair of diapers and any one of front and rear ends of the urine sensor extends beyond a front or a rear end of the paired diaper. The urine sensor extending beyond the associated end of the paired diaper is connected to an electric connector and adapted to be capable of sensing the wearer's urination.

### PRIOR ART DOCUMENT

### PATENT DOCUMENT

PATENT DOCUMENT 1: JP 2004-41697 A

US 2007/0038194 A1 and EP 1616542 A1 are considered to represent the closest prior art, they disclose urine receivers wherein urine discharged from a wearer is suctioned into a urine tank.

US 2007/083174 A1 relates to a method for detecting the presence of an insult in an absorbent article while it is worn by a wearer.

### SUMMARY OF THE INVENTION

### PROBLEM TO BE SOLVED BY THE INVENTION

The electric connector has, for example, an outer shape made of resinous material and a thickness dimension thereof is often larger than that of the urine sensor. The electric connector, extending beyond the associated end of the paired diaper, necessarily comes in direct contact with the wearer's skin and may cause any type of skin trouble. Furthermore, the relatively large thickness dimension of the electric connector may create a feeling of discomfort against the wearer.

It is an object of the present invention to provide an excretion sensing device improved so as not to create a feeling of discomfort against the wearer and any skin trouble to the wearer due to an electric connector to electrically connect electrodes to an excretion sensor including electrodes.

### MEASURE TO SOLVE THE PROBLEM

The present invention provides the excretion sensing device of independent claim 1. The dependent claims specify preferred but optional features.

According to the present invention, there is provided an excretion sensing device having a longitudinal direction and a transverse direction, a first end located on one of the ends as viewed in the longitudinal direction and a second end opposite to the first end and an upper surface and a lower surface, the excretion sensing device comprising an excretion receiver having an excretion sensor adapted to be connected to an exterior instrument via an electric connecter wherein the external instrument comprises at least an excretion vacuum pump to draw excretion.

The present invention is characterized in that the sensor comprises electrodes extending from the vicinity of the first end to the vicinity of the second end, wherein the electrodes are exposed from the lower surface in the vicinity of the first end to electrically and detachably connect to the connector.

According to the present invention, the sensor is partially exposed on the outer side facing the wearer's garment and the connector is adapted to be connected to the sensor on the outer side facing the wearer's garment.

With such unique arrangement, it can be avoided to bring the connector in direct contact with the wearer's body and does not therefore create a feeling of discomfort against the wearer's body.

The present invention may include preferred embodiments described below.

The embodiment of the present invention wherein a part of the sensor on which the electrodes are located extends outward of the receiver via a slit formed on the lower surface of the receiver. With the embodiment, an end of the sensor can be easily extended outward from the lower surface of the receiver.

The embodiment of the present invention wherein the sensor is provided with a first electrode and a second electrode which constitute the electrodes extending in the longitudinal direction and lying upon an elongate insulate sheet extending in the longitudinal direction and the electrodes are spaced from each other in the transverse direction. With the embodiment, the electrodes lie upon the elongate insulate sheet with a relative thickness, the electrodes do not create a feeling of discomfort against the wearer's body.

The embodiment of the present invention wherein the device further comprises an excretion sensing structure including a filter, spacer, the sensor and a cushion sheet located in order of distance from the upper surface toward the lower surface, respectively, wherein the sensor is sandwiched between the spacer and the cushion sheet. With the embodiment, the sensor is protected against the external impact even when a load of the wearer's body weight exerts the receiver, and the sensor does not produce improper operation signals.

The embodiment of the present invention wherein the sensing device further comprises a flexible and airtight excretion retainer comprising a bottom wall, a peripheral wall extending up from the bottom wall, a top wall, a first opening located in the inner space of the retainer and a second opening opened toward the outside of the retainer. With the embodiment, since the retainer is flexible, it does not create a feeling of discomfort against the wearer's body due to discomfortable stiffness of the retainer.

The embodiment of the present invention wherein the top wall is made of a breathability-resistant and liquid-pervious sheet. With the embodiment, it is ensured that the retainer allows excretion to permeate from the top wall into the inside of the retainer while keeping necessary airtightness thereof.

The embodiment of the present invention wherein the receiver is provided with projections extending up from the bottom wall and adapted to prevent the breathability-resistant and liquid-pervious sheet from sagging down into the inner space. With the embodiment, it is ensured that the retainer always maintains a definite capacity.

The embodiment of the present invention wherein the second opening is adapted to connect to the external instrument via a joint. With the embodiment, the receiver can be replaced with a fresh receiver as necessary and only the receiver may be used without using the external instrument.

The embodiment of the present invention wherein the moisture-absorbent structure comprises a moisture-pervious sheet forming an upper surface thereof, a liquid-impervious sheet forming the lower surface thereof and a moisture-absorbent material sandwiched between these sheets.

With the embodiment in paragraph 16, for example, where the receiver is applied to a wearing article, the moisture-absorbent structure serves to keep the inside of the wearing article comfortable.

The embodiment of the present invention wherein the moisture-absorbent structure has a concave region extending substantially along a center line of the retainer wherein the retainer may be set in the concave region.

The embodiment of the present invention wherein the concave region is devoid of a moisture-absorbent material and has a size corresponding to the size of the retainer in the longitudinal direction as well as in the transverse direction.

With the embodiments in paragraphs 18 and 19, the retainer does not create a feeling of discomfort against the wearer's body due to projection of the retainer from the top surface of the moisture-absorbent structure.

The embodiment of the present invention wherein the receiver is located on a chassis comprising a front waist region, a rear waist region, a crotch region extending between the front and rear regions, and the receiver extends in the longitudinal direction on the front waist region and the crotch region. With the embodiment, it is convenient for the wearer to apply the receiver to her or his body.

### BRIEF DESCRIPTION OF THE DRAWINGS

[FIG. 1] Fig. 1 is a diagram schematically illustrating a principle of the invention.
[FIG. 2] Fig. 2 is a plan view of an excretion receiver as viewed from a side of the wearer's body.
[FIG. 3] Fig. 3 is a sectional view taken along the line III-III in Fig. 2.
[FIG. 4] Fig. 4 is a sectional view taken along the line IV-IV in Fig. 2.
[FIG. 5] Fig. 5 is a plan view of the receiver as viewed from a side of the wearer's garment.
[FIG. 6] Fig. 6 is a perspective view of an excretion retainer.

### IDENTIFICATION OF REFERENCE NUMERALS USED IN THE DRAWINGS

- 1: wearing article
- 2: chassis
- 3: excretion receiver
- 4: excretion sensing structure
- 5: excretion retainer
- 6: moisture-absorbent structure
- 21: front waist region
- 22: rear waist region
- 23: crotch region
- 41: excretion sensor
- 45a: first electrode
- 45b: second electrode
- 48: electric connector
- 65: concave region
- C: control unit
- P: excretion vacuum pump
- T: excretion tank

### DETAILED DESCRIPTION OF THE PREFERRED EMBODIMENTS

Details of the present invention will be more fully understood from the description given hereunder on the basis of an exemplary embodiment of a pant-shaped wearing article comprising a chassis.

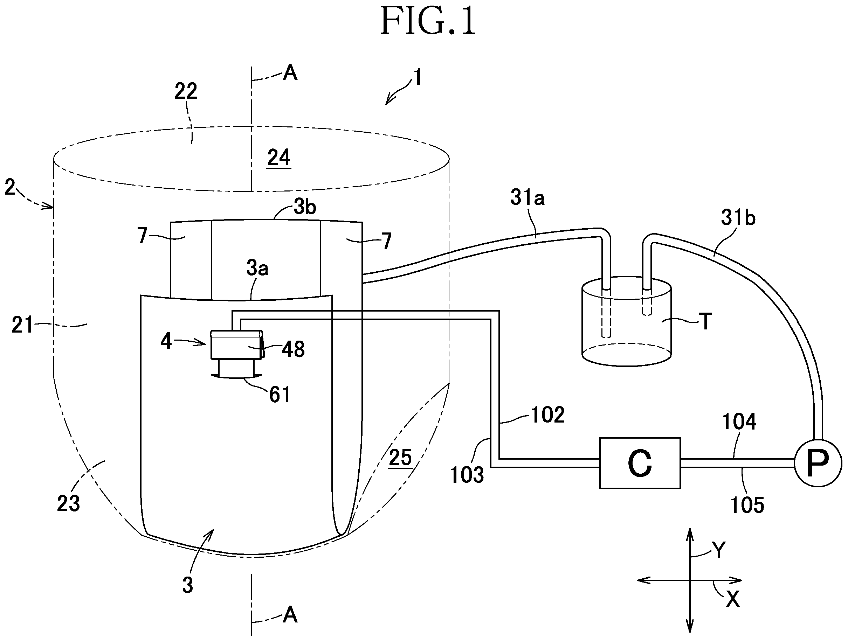

Fig. 1 is a diagram schematically illustrating a wearing article 1 including an excretion sensing device according to the invention and an external instrument to which the wearing article 1 connects as a whole.

According to the present invention, the wearing article 1 is provided on its pant-shaped chassis with an excretion receiver 3 which is coupled to an excretion tank T via a tube 31a, wherein the tank T is coupled to a vacuum pump P via a tube 31b. The pump P is coupled to a control unit C via the tube 31b. When an excretion sensing structure 4 senses excretion (particularly urine, hereunder same), a sensing signal is impressed to the control unit C, then an electric motor (not shown) is activated so that the pump P begins operation to draw excretion out of the article 1. Upon actuation of the pump T, the tank T creates a vacuum so that excretion received in the receiver 3 will be drawn into the tank T. Such kinds of excretion sensing systems or excretion drawing systems themselves are known in this field of art. With reference to the present invention, the control unit C, tank T and pump P are collectively referred to as an external instrument.

The chassis 2 has a wearer's body side (hereinafter, may be referred to as upper side) and an opposite side thereof (hereinafter, may be referred to as lower side) and comprises a front waist region 21, a rear waist region 22 and a crotch region 23 extending between the front and rear waist regions 21, 22 as viewed in a longitudinal direction Y. The chassis 2 is formed of a mesh-textured sheet made of an elastic material such as elastic yarns and elasticized in the longitudinal direction Y as well as in a transverse direction X being orthogonal to the longitudinal direction Y. The chassis 2 has a sufficiently high elastic force particularly around a waist-opening 24 and a pair of leg-openings 25 so that the chassis 2 may be kept in close contact with a waist and legs of the wearer's body.

The chassis 2 is provided on its inner side facing the wearer's body with the receiver 3 extending across the front waist region 21, the crotch region 23 and the rear waist region 22 in the longitudinal direction Y along a longitudinal center line A-A bisecting a dimension of the chassis 2 as measured in the transverse direction X. The receiver 3 is held under the elastic force of the chassis 2 in close contact with the wearer' s body and exchangeable independently of the chassis 2.

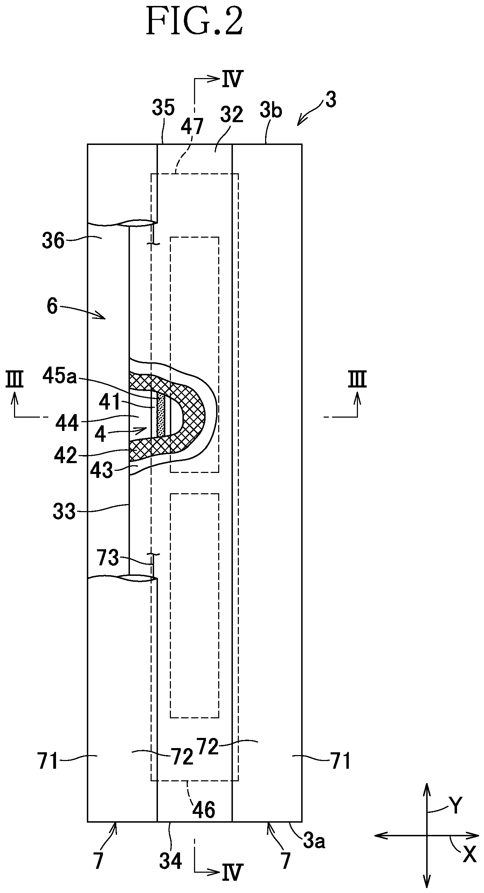

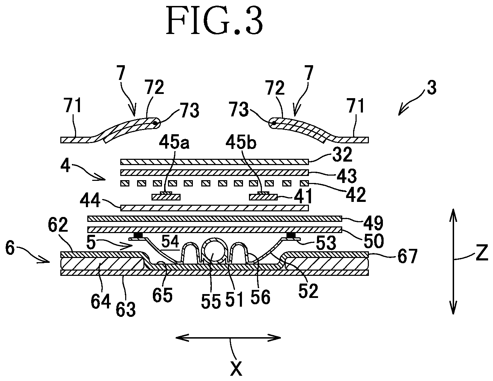

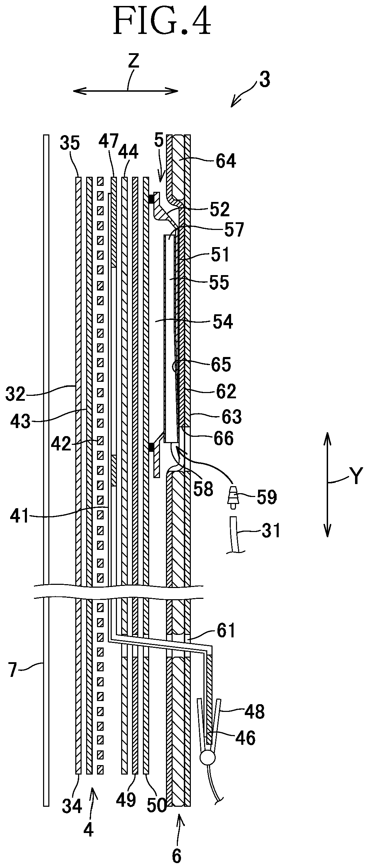

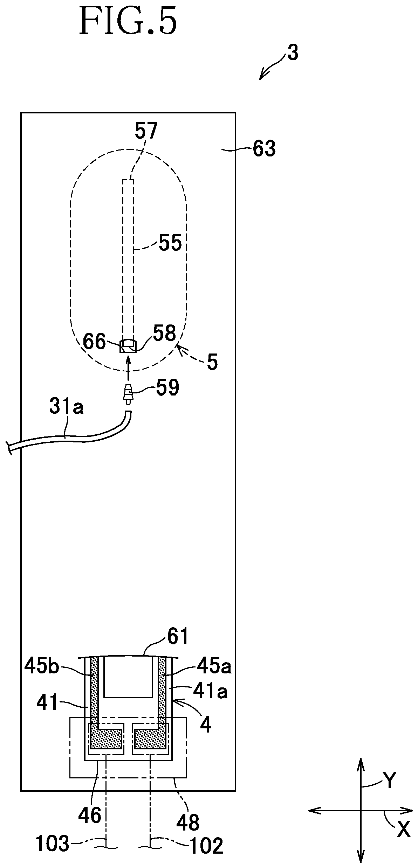

Fig. 2 is a plan view showing the receiver 3 as flatly developed and viewed from the inner side facing the wearer's body. In Fig. 2, the receiver 3 is shown as partially cutaway for convenience of illustration and the chassis 2 is eliminated. Fig. 3 is a sectional view taken along the line III-III in Fig. 2 and Fig. 4 is a sectional view taken along the line IV-IV in Fig. 2. While respective component sheets are separated one from another for convenience of illustration in Figs. 3 and 4, it should be appreciated that these component sheets are actually joined together. Fig. 5 is a plan view showing the same receiver 3 as that shown in Fig. 2 as viewed from the lower side.

The receiver 3 has a first end 3a located on the front waist region 21 of the chassis 2, a second end 3b located on the rear waist region 22 of the chassis 2 and a vertical direction (thickness direction) Z, and comprises a top-sheet 32 to come in contact with the wearer's body, an excretion sensing structure 4, a tray-shaped excretion retainer 5, and a moisture-absorbent structure 6 to absorb moisture inside the chassis 2.

The top-sheet 32 may be made, for example, of a liquid-pervious fibrous non-woven fabric. The top-sheet 32 is shaped generally in a rectangle extending in the longitudinal direction Y and contoured by a pair of side edges 33, 33 extending in the longitudinal direction Y and a pair of ends (i.e., front and rear ends) 34, 35 extending in the transverse direction X.

A pair of barrier cuffs 7, 7 against excretion are overlaid on the top-sheet 32. The barrier cuffs 7, 7 respectively include proximal edges 71, 71 lying outward as viewed in the transverse direction X and distal edges 72, 72 lying inward with respect to the proximal edges 71, 71 also as viewed in the transverse direction X. The respective proximal edges 71, 71 extend outward beyond the associated side edges 33, 33 of the top-sheet 32 as viewed in the transverse direction X and are joined to the moisture-absorbent structure 6. The distal edges 72, 72 are free to be spaced upward from the top-sheet 32 toward the wearer's body. The distal edges 72, 72 are elasticized by cuff elastic members 73, 73 attached under tension thereto so that, upon contraction of the elastic members 73, 73, the distal edges 72, 72 are spaced upward from the top-sheet 32 toward the wearer's body, thereby preventing excretion from leaking outward in the transverse direction X. As a stock material for the barrier cuffs 7, 7, it is possible to use a fibrous non-woven fabric or a porous plastic film which is sufficient liquid-impervious to prevent excretion leakage but vapor-pervious.

The sensing structure 4 extends in the longitudinal direction across the front waist region 21, the crotch region 23 and the rear waist region 22. The sensing structure 4 comprises an excretion sensor 41, a spacer 42, a filter 43 and a cushion sheet 44. With such an arrangement, the sensor 41 is sandwiched between the spacer 42 and the cushion sheet 44 in the thickness direction Z and protected against any external impact.

The control unit C is adapted to electrically connect to the sensor 41 via an electric connector 48 attached to ends of electric leads 102, 103 extending from the control unit C. In addition, the control unit C is electrically connected to a electric motor (not shown) for activating the pump P via electric leads 104, 105. Upon sensing of excretion by the sensor 41, a sensing signal is impressed to the control unit C, then the control unit C is activated to drive the motor of the pump P. The pump P, in turn, draws air inside the tank T via the tube 31b. Consequently, the tank T creates a vacuum, so that excretions are drawn out from the receiver 3 via the tube 31a into the tank T and pooled therein.

The sensor 41 has first and second ends 46, 47 respectively in the vicinity of the first and second ends 3a, 3b of the receiver 3 extending in the transverse direction X. The sensor 41 comprises an insulate base sheet 41a forming first and second electrodes 45a, 45b extending in longitudinal direction and spaced from each other in the transverse direction X. The base sheet 41a is made of a thermoplastic synthetic film such as a polyethylene film and is dimensioned to be shorter than the dimension of the chassis 2 as viewed in the longitudinal direction Y. The formation of the first and second electrodes 45a, 45b on the base sheet 41a may be formed by coating an electrically-conductive coating material, for example, using an appropriate printing technique. The first and second electrodes 45a, 45b are adapted to electrically connect each other via excretion when the excretion flow into between them.

The spacer 42 is made of a mesh-textured sheet having high air-permeability and liquid-permeability and serves to keep the filter 43 and the sensor 41 spaced from each other in the thickness direction Z. Should the sensor 41 be held in contact with the filter 43 in a wet state for an excessive period, the sensor 41 may be kept energized, causing malfunction sensing of excretion without occurrence of excretion. Even when the wearer's body weight exerted on the receiver 3, the presence of the spacer 42 prevents the filter 43 and the sensor 41 from being held in contact with each other.

Below the cushion sheet 44, there are provided a dispersant sheet 49 and a breathability-resistant and liquid-permeable sheet 50 that are laminated in this order. Below the breathability-resistant sheet 50, the retainer 5 is provided. The breathability-resistant sheet 50 and the retainer 5 are bonded to each other by any appropriate bonding means such as an adhesive agent. The breathability-resistant and liquid-permeable sheet 50 preferably has air-permeability of 0 - 100cc/cm²/sec in a wet state and of 20 - 200cc/cm²/sec in a dry state.

The dispersant sheet 49 allows discharged excretion to disperse over the breathability-resistant sheet 50 and thereby to wet this breathability-resistant sheet 50 extensively and rapidly. As a stock material for the dispersant sheet 49, a non-woven fabric containing hydrophilic fibers such as rayon fibers may be used. The breathability-resistant and liquid-permeable sheet 50 is high in liquid-permeability but low in breathability and, as a stock material for this sheet, for example, a fibrous non-woven fabric modified to become hydrophilic may be used.

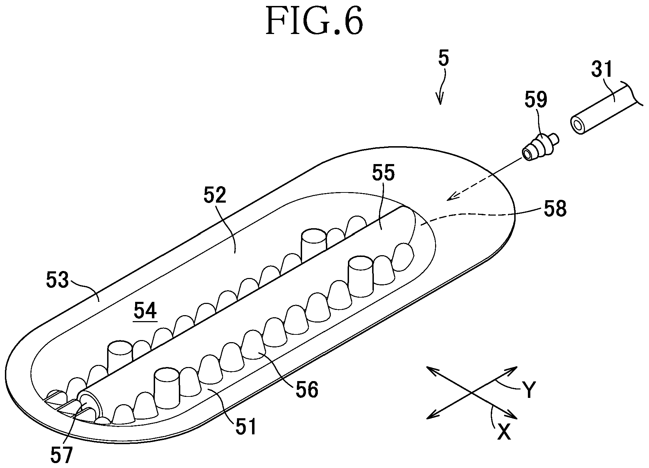

The retainer 5 is flexible and may be made of a soft and liquid-impervious elastic material such as soft polyethylene or silicon rubber. The retainer 5 may be easily made, for example, by known injection molding machines. Fig. 6 is a perspective view of the retainer 5. As shown, the retainer 5 comprises a bottom wall 51, a peripheral wall 52 extending up from the bottom wall 51 and a flange 53 extending outward from a top of the peripheral wall 52. The flange 53 is held in contact with the breathability-resistant and liquid-permeable sheet 50 to define a space 54 within the retainer 5. The retainer 5 is formed within it with a vacuum chamber 55 functioning to collect excretion flowing thereinto and then to discharge this out. The chamber 55 has a cylindrical shape and extends in the longitudinal direction of the retainer 5 substantially along a center line of the retainer 5 as viewed in the transverse direction X thereof. On each side of the chamber 55 in the transverse direction X thereof, an array of projections 56 extends upward from the bottom wall 51 toward the wearer's body. The projections 56 serve to prevent the breathability-resistant and liquid-permeable sheet 50 held in contact with the flange 53 from further sagging down into the space 54.

As will be apparent from Figs. 5 and 6, a first opening 57 which is one end of the chamber 55 is opened within the retainer 5 and a second opening 58 which is the other end of the chamber 55 is opened toward the exterior of the retainer 5. The tube 31a is attached to the second opening 58 of the chamber 55 via a joint 59 and the tube 31a is coupled to the pump P.

As will be apparent from Figs. 3 and 4, the moisture-absorbent structure 6 is provided below the retainer 5. The moisture-absorbent structure 6 comprises a moisture-pervious sheet 62 facing the retainer 5, a liquid-impervious sheet 63 and a moisture-absorbent material 64 sandwiched between these sheets 62, 63. The moisture-absorbent material 64 comprises, for example, a mixture of fluff pulp, super-absorbent particles (SAP) and silica gel as a moisture-absorbent core wrapped with tissue paper or the like. Such moisture-absorbent structure 6 has a concave region 65 extending in the longitudinal direction Y substantially along a center line of the retainer 5 as viewed in the transverse direction X, wherein the retainer 5 may be set in the concave region 65. The concave region 65 is devoid of the moisture-absorbent material 64 as viewed in its thickness direction and has a size corresponding to the size of the retainer 5 in the longitudinal direction Y as well as in the transverse direction X. As a consequence, the moisture-absorbent structure 6 has its thickness correspondingly reduced in the concave region 65 in which the moisture-pervious sheet 62 and the liquid-impervious sheet 63 are directly put flat together. The retainer 5 may fit into this concave region 65. As an advantageous effect of such design, a dimension of the moisture-absorbent structure 6 as measured in the thickness direction is substantially uniform, regardless of the retainer 5 being present or not on the moisture-absorbent structure 6, and a feeling of discomfort against the wearer' s body which otherwise should be experienced by the wearer due to the presence of the retainer 5 can be reduced. In addition, since the retainer 5 is not in sharp contact with the wearer's body, this design can prevent the wearing article from hurting the wearer.

With reference to Fig. 6 again, the second opening 58 of the chamber 55 is exposed from a window 66 formed in the moisture-absorbent structure 6. More specifically, the window 66 is formed in the concave region 65 in which the moisture pervious sheet 62 is kept in direct contact with the liquid-impervious sheet 63. In other words, the window 66 may be formed by piercing through only these two sheets 62, 63 and it is unnecessary to pierce through the moisture-absorbent material 64. It is correspondingly easier to form the window 66 than the case in which also the moisture-absorbent material 64 must be pierced through.

The moisture-absorbent structure 6 is dimensioned in the transverse direction X to be larger than those of both the receiver 3 and the sensing structure 4. In other words, the moisture-absorbent structure 6 extends in the transverse direction X beyond the respective opposite side edges of the receiver 3 and the sensing structure 4, and the proximal edges 71, 71 of the respective barrier cuffs 7, 7 are bonded to the moisture-pervious sheet 62 of the moisture-absorbent structure 6 in regions thereof extending beyond the receiver 3 and the sensing structure 4.

The article with such construction operates in response to excretion in a manner as will be described hereinafter. Upon occurrence of excretion, the excretion permeates the top-sheet 32. In the course of permeation, any amount of excretion should not leak out from the article in the transverse direction X since the top-sheet 32 is provided along the side edges 33, 33 with the barrier cuffs 7, 7, respectively.

The excretion having permeated the top-sheet 32 rapidly passes through the filter 43 and the spacer 42 and comes in contact with the sensor 41. Upon continuous contact with the excretion, the first and second electrodes 45a, 45b are electrically connected to each other via the excretion. The electrical connection causes a sensing circuit to be closed and electrified so that the excretion can be sensed. It should be noted here that a voltage from a power source (not shown) is always applied to the circuit. The control unit C transmits a signal impressing the sensing of the excretion to the motor of the pump P and activates the pump P. Upon actuation of the pump P, the tank T creates a vacuum so that the excretion pooled within the space 54 of the retainer 5 is drawn out into the tank T via the tube 31a. In this way, the excretion within the space 54 can be discharged from the chassis 2. After the excretion is discharged from the chassis 2 and exists between the first and second electrodes 45a and 45b no more, the sensing circuit opens again. By the opening of the sensing circuit and the ending of the electrification, transmittance of the signal to the electric motor of the pump P is ended and then pump P stops.

The excretion having moved to the sensor 41 then permeates the dispersant sheet 49 through the cushion sheet 44. The excretion is dispersed in the dispersant sheet 49 over a wide range and then permeates the breathability-resistant and liquid -permeable sheet 50, thereby to make the space 54 of the retainer 5. Therefore the space 54 creates a negative pressure under the effect of the pump P. As a consequence, the excretion saturating the breathability-resistant and liquid-permeable sheet 50 is drawn into the space 54 and the excretion having been drawn into the space 54 in this manner is then drawn out via the chamber 55 to the exterior of the receiver 5. In this way, the excretion can be expelled out from the wearing article in a short time after occurrence of excretion.

Theoretically, the wearing article is substantially free from any amount of excretion after the excretion has been expelled out under drawing by the pump P. However, a slight amount of excretion may remain without being drawn, particularly on the retainer 5. Since this retainer 5 is liquid-impervious, the excretion likely remains on the surface of the retainer 5. The excretion remaining in the article without being drawn is vaporized under the effect of a body temperature of the wearer and filled in the wearing article 1 in the form of vapor. However, such vapor is reliably absorbed by the moisture-absorbent structure 6 provided below the receiver 3.

Specifically, such vapor is absorbed through the moisture-pervious sheet 62 of the moisture-absorbent structure 6 into the moisture-absorbent material 64. The liquid-impervious sheet 63 provided below the moisture-absorbent material 64 reliably confines such vapor within the moisture absorbent material 64. In this way, the humidity within the wearing article 1 should not increase and any skin trouble or a feeling of discomfort due to increased humidity should be experienced by the wearer can be restricted.

In the wearing article 1 as exemplarily illustrated, the connector 48 is provided below the moisture-absorbent structure 6 and therefore the connector 48 should not come in direct contact with the wearer's body. As an advantageous consequence, the wearer's skin is protected against discomfortable stiffness and/or rash due to direct contact with the connector 48, possibly leading to various types of skin trouble. With such unique arrangement, wherein the connector 48 can be located below the moisture-absorbent structure 6, the moisture-absorbent structure 6 functions as a cushion adapted to protect the wearer against hurting due to the connector 48 being directly pressed against the wearer's skin and/or a feeling of discomfort due to the presence of the connector 48.

While the concave region 65 of the moisture-absorbent structure 6 is formed by locally removing the moisture-absorbent material 64 fully in the thickness direction so far as the illustrated exemplary embodiment is concerned, it is not essential to remove the moisture-absorbent material 64 fully in the thickness direction and the effect of the invention is not significantly affected by any amount of the moisture-absorbent material 64 remaining between the moisture-pervious sheet 62 and the liquid-impervious sheet 63.

While the exemplary embodiment of the invention has been described above to have the sensor 41 adapted to sense excretion exclusively, the invention may be exploited as the article additionally having a sensor exclusively for defecation. While the exemplary embodiment uses the mesh-textured pant as the chassis, it is possible to use the other type of chassis selected from a wide range of members such as disposable diapers, diaper covers, T-shaped belts and the like. Though the receiver 3 is previously attached to the chassis 2 in the illustrated embodiment, only the receiver can be sold without the chassis.

It should be noted that the electric connector 48 has the known configuration of a clip which can detachably hold the first end 46 of the sheet-like sensor 41 and is provided inside thereof with electric terminals adapted to electrically connect to the first and second electrodes 45a, 45b.

The receiver 3 may be formed, for example, by the following method.

The retainer 5, of which the top surface is covered with the breathability-resistant and liquid-permeable sheet 50, is set in the concave region 65 of the moisture-absorbent structure 6 including the moisture-permeable sheet 62, the liquid-impermeable sheet 63 and the moisture-absorbent material 64, and then the dispersant sheet 49, the sensing structure 4 including the sensor 41, the spacer 42, the filter 43,the cushion sheet 44 and the top sheet 32 are layered above the retainer 5 in an upward order. Finally, the barrier cuffs 7, 7 are attached on the sides of the moisture-absorbent structure 6. It should be noted here that materials of elements/members and structure of the receiver 3 have been already described. They are bonded or joined, for example, using well-known adhesives or heat-sealing means.

## Claims

1. An excretion sensing device having a longitudinal direction (Y) and a transverse direction (X), a first end (3a) located on one of the ends as viewed in said longitudinal direction and a second end (3b) opposite to said first end and an upper surface and a lower surface, the excretion sensing device comprising an excretion receiver (3) having an excretion sensor (41) adapted to be electrically connected to an exterior instrument via an electric connecter (48), said external instrument comprising at least an excretion vacuum pump (P) to draw excretion therein, wherein
the excretion receiver (3) comprises an excretion sensing structure (4), located between said upper and lower surfaces of the excretion sensing device, and an airtight excretion retainer (5) located below said excretion sensing structure (4) ; wherein
said excretion sensor (41) comprises electrodes (45a, 45b) extending from the vicinity of said first end (3a) to the vicinity of said second end (3b), **characterised in that** said electrodes (45a, 45b) are exposed from said lower surface in the vicinity of said first end (3a) to electrically and detachably connect to said connector (48);
said excretion receiver (3) further comprises a moisture-absorbent structure (6) below said airtight excretion retainer (5); and
said moisture absorbent structure (6) has a concave region (65) extending in said longitudinal direction (Y), and said airtight excretion retainer (5) is set in said concave region (65).

2. The device according to Claim 1, wherein a part of said sensor (41) on which said electrodes (45a, 45b) are located extends outward of said receiver (3) via a slit formed on said lower surface of said receiver (3).

3. The device according to Claim 1 or 2, wherein said sensor (41) is provided with a first electrode (45a) and a second electrode (45b) which constitute said electrodes (45a, 45b) extending in said longitudinal direction (Y) and lying upon an elongate insulate sheet (41a) extending in said longitudinal direction and said electrodes (45a, 45b) are spaced from each other in said transverse direction (X).

4. The device according to any one of Claims 1 through 3, wherein said excretion sensing structure (4) comprises a filter (43), a spacer (42), said sensor (41) and a cushion sheet (44) located in order of distance from said upper surface toward said lower surface, and said sensor (41) is sandwiched between said spacer (42) and said cushion sheet (44).

5. The device according to any one of claims 1 though to 4, wherein said airtight excretion retainer (5) comprises a bottom wall (51), a peripheral wall (52) extending up from said bottom wall (51), a top wall, a first opening (57) located in said inner space (54) of said retainer (5) and a second opening (58) opened toward the outside of said retainer (5).

6. The device according to Claim 5, wherein said top wall is made of a breathability-resistant and liquid-pervious sheet (50).

7. The device according to Claim 5 or 6, wherein said receiver (3) is provided with projections (56) extending up from said bottom wall (51) and adapted to prevent said breathability-resistant and liquid-pervious sheet (50) from sagging down into said inner space (54).

8. The device according to Claim 5 or 6, wherein said second opening (58) is adapted to connect to said external instrument via a joint (59).

9. The device according to Claim 1, wherein said moisture-absorbent structure (6) comprises a moisture-pervious sheet (62) forming said upper surface thereof, a liquid-impervious sheet (63) forming said lower surface thereof and a moisture-absorbent material (64) sandwiched between these sheets (62, 63).

10. The device according to Claim 1 or 9, wherein the concave region (65) of said moisture-absorbent structure (6) extends in said longitudinal direction substantially along a center line (A) of said retainer.

11. The device according to Claims 1, 9 or 10, wherein said concave region (65) is devoid of a moisture-absorbent material and has a size corresponding to the size of said retainer (5) in said longitudinal direction (Y) as well as in said transverse direction (X).

12. The device according to any one of Claims 1 through 11, wherein said receiver (3) is located on a chassis (22) comprising a front waist region (21), a rear waist region (22), a crotch region (23) extending between said front and rear regions, and said receiver (3)extends in said longitudinal direction on said front waist region (21) and said crotch region (23).

## Patentansprüche

1. Ausscheidungssensorvorrichtung mit einer Längsrichtung (Y) und einer Querrichtung (X), einem ersten Ende (3a) an einem der in der besagten Längsrichtung betrachteten Enden und einem zweiten Ende (3b) gegenüber dem besagten ersten Ende und einer oberen Oberfläche und einer unteren Oberfläche, wobei die Ausscheidungssensorvorrichtung einen Ausscheidungssammler (3) mit einem Ausscheidungssensor (41) umfasst, der für elektrische Verbindung mit einem Außeninstrument über einen elektrischen Verbinder (48) ausgelegt ist, wobei das besagte Außeninstrument mindestens eine Ausscheidungs-Vakuumpumpe (P) zum Ansaugen von Ausscheidungen umfasst, wobei
der Ausscheidungssammler (3) eine zwischen den besagten oberen und unteren Oberflächen der Ausscheidungssensorvorrichtung angeordnete Ausscheidungssensorstruktur (4) und einen unterhalb der besagten Ausscheidungssensorstruktur (4) angeordneten luftdichten Ausscheidungshalter (5) umfasst; wobei
der besagte Ausscheidungssensor (41) Elektroden (45a, 45b) umfasst, die sich von der Nähe des besagten ersten Endes (3a) zur Nähe des besagten zweiten Endes (3b) erstrecken,
**dadurch gekennzeichnet, dass** die besagten Elektroden (45a, 45b) zur lösbaren elektrischen Verbindung mit dem besagten Verbinder (48) von der besagten unteren Oberfläche aus in der Nähe des besagten ersten Endes (3a) freiliegen;
dass der besagte Ausscheidungssammler (3) weiter eine Feuchtigkeit aufnehmende Struktur (6) unter dem besagten luftdichten Ausscheidungshalter (5) umfasst; und
dass die besagte Feuchtigkeit aufnehmende Struktur (6) einen sich in der besagten Längsrichtung (Y) erstreckenden konkaven Bereich (65) aufweist und der besagte luftdichte Ausscheidungshalter (5) in dem besagten konkaven Bereich (65) angeordnet ist.

2. Vorrichtung nach Anspruch 1, wobei ein Teil des besagten Sensors (41), wo die besagten Elektroden (45a, 45b) angeordnet sind, sich von dem besagten Sammler (3) aus über einen in der besagten unteren Oberfläche des besagten Sammlers (3) ausgebildeten Schlitz nach außen erstreckt.

3. Vorrichtung nach Anspruch 1 oder 2, wobei der besagte Sensor (41) mit einer ersten Elektrode (45a) und einer zweiten Elektrode (45b) ausgestattet ist, die die besagten Elektroden (45a, 45b) bilden, die sich in der besagten Längsrichtung (Y) erstrecken und auf einer länglichen Isolierlage (41 a) liegen, die sich in der besagten Längsrichtung erstreckt, und wobei die besagten Elektroden (45a, 45b) in der besagten Querrichtung (X) voneinander beabstandet sind.

4. Vorrichtung nach einem der Ansprüche 1 bis 3, wobei die besagte Ausscheidungssensorstruktur (4) einen Filter (43), ein Distanzstück (42), den besagten Sensor (41) und eine Kissenlage (44) umfasst, die in Reihenfolge ihrer Entfernung von der besagten oberen Oberfläche in Richtung der besagten unteren Oberfläche angeordnet sind, und wobei der besagte Sensor (41) zwischen dem besagten Distanzstück (42) und der besagten Kissenlage (44) angeordnet ist.

5. Vorrichtung nach einem der Ansprüche 1 bis 4, wobei der besagte luftdichte Ausscheidungshalter (5) eine Bodenwand (51), eine sich von der besagten Bodenwand (51) aus erstreckende Umfangswand (52), eine obere Wand, eine im besagten Innenraum (54) des besagten Halters (5) angeordnete erste Öffnung (57) und eine in Richtung der Außenseite des besagten Halters (5) geöffnete zweite Öffnung (58) umfasst.

6. Vorrichtung nach Anspruch 5, wobei die besagte obere Wand aus einer nichtatmungsaktiven und flüssigkeitsdurchlässigen Lage (50) besteht.

7. Vorrichtung nach Anspruch 5 oder 6, wobei der besagte Sammler (3) mit Vorsprüngen (56) versehen ist, die sich von der besagten Bodenwand (51) aus nach oben erstrecken und so ausgebildet sind, dass sie das Absinken der besagten nicht-atmungsaktiven und flüssigkeitsdurchlässigen Lage (50) in den besagten Innenraum (54) verhindern.

8. Vorrichtung nach Anspruch 5 oder 6, wobei die besagte zweite Öffnung (58) zur Verbindung mit dem besagten Außeninstrument über ein Verbindungsstück (59) ausgelegt ist.

9. Vorrichtung nach Anspruch 1, wobei die besagte Feuchtigkeit aufnehmende Struktur (6) eine ihre besagte obere Oberfläche bildende feuchtigkeitsdurchlässige Lage (62), eine ihre besagte untere Oberfläche bildende flüssigkeitsundurchlässige Lage (63) und ein zwischen diesen Lagen (62, 63) angeordnetes Feuchtigkeit aufnehmendes Material (64) umfasst.

10. Vorrichtung nach einem der Ansprüche 1 bis 9, wobei der konkave Bereich (65) der besagten Feuchtigkeit aufnehmenden Struktur (6) sich in der besagten Längsrichtung im Wesentlichen entlang einer Mittellinie (A) des besagten Halters erstreckt.

11. Vorrichtung nach Anspruch 1, 9 oder 10, wobei der besagte konkave Bereich (65) kein Feuchtigkeit aufnehmendes Material aufweist und eine Größe hat, die in der besagten Längsrichtung (Y) sowie in der besagten Querrichtung (X) der Größe des besagten Halters (5) entspricht.

12. Vorrichtung nach einem der Ansprüche 1 bis 11, wobei der besagte Halter (3) an einem Rahmen (22) angeordnet ist, der aus einem vorderen Taillenbereich (21), einem rückwärtigen Taillenbereich (22) und einem sich zwischen dem vorderen und dem rückwärtigen Taillenbereich erstreckenden Schrittbereich (23) umfasst, und wobei sich der besagte Halter (3) in der besagten Längsrichtung des besagten vorderen Taillenbereichs (21) und des besagten Schrittbereichs (23) erstreckt.

## Revendications

1. Dispositif de détection d'excrétion ayant une direction allant dans le sens longitudinal (Y) et une direction allant dans le sens transversal (X), une première extrémité (3a) se trouvant sur l'une des extrémités quand le dispositif est vu dans ladite direction allant dans le sens longitudinal et une deuxième extrémité (3b) opposée par rapport à ladite première extrémité et une surface supérieure et une surface inférieure, le dispositif de détection d'excrétion comportant un dispositif de réception d'excrétion (3) ayant un capteur d'excrétion (41) adapté à des fins de connexion électrique à un instrument extérieur par le biais d'un connecteur électrique (48), ledit instrument externe comportant au moins une pompe à vide d'excrétion (P) servant à aspirer toute excrétion dans celle-ci, dans lequel
le dispositif de réception d'excrétion (3) comporte une structure de détection d'excrétion (4), se trouvant entre lesdites surfaces supérieure et inférieure du dispositif de détection d'excrétion, et un dispositif de retenue d'excrétion étanche à l'air (5) se trouvant en dessous de ladite structure de détection d'excrétion (4) ; dans lequel
ledit capteur d'excrétion (41) comporte des électrodes (45a, 45b) s'étendant depuis la région proche de ladite première extrémité (3a) jusqu'à la région proche de ladite deuxième extrémité (3b), **caractérisé en ce que**
lesdites électrodes (45a, 45b) sont exposées depuis ladite surface inférieure dans la région proche de ladite première extrémité (3a) à des fins de connexion électrique et détachable audit connecteur (48) ;
ledit dispositif de réception d'excrétion (3) comporte par ailleurs une structure d'absorption d'humidité (6) en dessous dudit dispositif de retenue d'excrétion étanche à l'air (5) ; et
ladite structure d'absorption d'humidité (6) a une région concave (65) s'étendant dans ladite direction allant dans le sens longitudinal (Y), et ledit dispositif de retenue d'excrétion étanche à l'air (5) est installé dans ladite région concave (65).

2. Dispositif selon la revendication 1, dans lequel une partie dudit capteur (41), sur lequel se trouvent lesdites électrodes (45a, 45b), s'étend vers l'extérieur par rapport audit dispositif de réception (3) par le biais d'une fente formée sur ladite surface inférieure dudit dispositif de réception (3).

3. Dispositif selon la revendication 1 ou la revendication 2, dans lequel ledit capteur (41) comporte une première électrode (45a) et une deuxième électrode (45b) qui constituent lesdites électrodes (45a, 45b) s'étendant dans ladite direction allant dans le sens longitudinal (Y) et reposant sur une feuille d'isolation allongée (41a) s'étendant dans ladite direction allant dans le sens longitudinal et lesdites électrodes (45a, 45b) sont espacées l'une par rapport à l'autre dans ladite direction allant dans le sens transversal (X).

4. Dispositif selon l'une quelconque des revendications 1 à 3, dans lequel ladite structure de détection d'excrétion (4) comporte un filtre (43), une pièce d'écartement (42), ledit capteur (41) et une feuille formant coussinet (44) se trouvant selon un ordre de distance depuis ladite surface supérieure vers ladite surface inférieure, et ledit capteur (41) est pris en sandwich entre ladite pièce d'écartement (42) et ladite feuille formant coussinet (44).

5. Dispositif selon l'une quelconque des revendications 1 à 4, dans lequel ledit dispositif de retenue d'excrétion étanche à l'air (5) comporte une paroi inférieure (51), une paroi périphérique (52) s'étendant vers le haut depuis ladite paroi inférieure (51), une paroi supérieure, une première ouverture (57) se trouvant dans ledit espace intérieur (54) dudit dispositif de retenue (5) et une deuxième ouverture (58) ouverte vers l'extérieur dudit dispositif de retenue (5).

6. Dispositif selon la revendication 5, dans lequel ladite paroi supérieure est réalisée à partir d'une feuille résistante à la respirabilité et perméable aux liquides (50).

7. Dispositif selon la revendication 5 ou la revendication 6, dans lequel le dispositif de réception (3) comporte des parties saillantes (56) s'étendant vers le haut depuis ladite paroi inférieure (51) et adaptées pour empêcher ladite feuille résistante à la respirabilité et perméable aux liquides (50) de s'affaisser vers le bas dans ledit espace intérieur (54).

8. Dispositif selon la revendication 5 ou la revendication 6, dans lequel ladite deuxième ouverture (58) est adaptée à des fins de connexion audit instrument externe par le biais d'un joint (59).

9. Dispositif selon la revendication 1, dans lequel la structure d'absorption d'humidité (6) comporte une feuille perméable à l'humidité (62) formant ladite surface supérieure de celle-ci, une feuille imperméable aux liquides (63) formant ladite surface inférieure de celle-ci et un matériau absorbant l'humidité (64) pris en sandwich entre ces feuilles (62, 63).

10. Dispositif selon la revendication 1 ou la revendication 9, dans lequel la région concave (65) de ladite structure d'absorption d'humidité (6) s'étend dans ladite direction allant dans le sens longitudinal sensiblement le long d'une ligne centrale (A) dudit dispositif de retenue.

11. Dispositif selon la revendication 1, la revendication 9 ou la revendication 10, dans lequel ladite région concave (65) est dépourvue de tout matériau absorbant l'humidité et a une taille correspondant à la taille dudit dispositif de retenue (5) dans ladite direction allant dans le sens longitudinal (Y) ainsi que dans ladite direction allant dans le sens transversal (X).

12. Dispositif selon l'une quelconque des revendications 1 à 11, dans lequel ledit dispositif de réception (3) se trouve sur une armature (22) comportant une région avant au niveau de la taille (21), une région arrière au niveau de la taille (22), une région au niveau de l'entrejambe (23) s'étendant entre lesdites régions avant et arrière, et ledit dispositif de réception (3) s'étend dans ladite direction allant dans le sens longitudinal sur ladite région avant au niveau de la taille (21) et ladite région au niveau de l'entrejambe (23).
